# EUROPÄISCHE PATENTSCHRIFT

(11) **EP 1 938 089 B1**
(45) Veröffentlichungstag und Bekanntmachung des Hinweises auf die Patenterteilung: **23.11.2016**
(21) Anmeldenummer: 06806259.5
(22) Anmeldetag: 13.10.2006
(51) Int. Cl.: G01N 21/952, G01N 21/88, B66B 7/12, D01H 13/26, G01N 33/36

(54) **VERFAHREN UND VORRICHTUNG ZUM INSPIZIEREN EINES LAUFENDEN DRAHTSEILS**
METHOD AND DEVICE FOR INSPECTING A TRAVELING WIRE CABLE
PROCEDE ET DISPOSITIF POUR INSPECTER UN CABLE METALLIQUE EN MOUVEMENT

(30) Priorität: 20.10.2005 DE 102005050220
(43) Veröffentlichungstag der Anmeldung: 02.07.2008
(73) Patentinhaber: Casar Drahtseilwerk Saar GmbH, 66459 Kirkel (DE)
(72) Erfinder: VERREET, Roland, 52072 Aachen (DE)
(74) Vertreter: Bernhardt, Reinhold
(86) Internationale Anmeldenummer: PCT/EP2006/009908
(87) Internationale Veröffentlichungsnummer: WO 2007/045403

(56) Entgegenhaltungen:
- EP-A2- 0 271 728
- EP-A2- 0 565 906
- DE-A1- 19 742 177
- US-A- 3 599 223
- US-A- 3 761 177
- WEHKING K-H ET AL: "INNOVATIVE PROCEDURE FOR VISUAL ROPE INSPECTION", LIFT REPORT, VFZ VERLAG, DORTMUNT, DE, vol. 29, no. 3, 1 January 2003 (2003-01-01), page 10,12,14, XP001162513, ISSN: 0341-3721

## Beschreibung

Die Erfindung betrifft ein Verfahren zum Inspizieren eines laufenden Drahtseils, bei dem das laufende Drahtseil an einer ortsfesten Stelle in zeitlichen Abständen mindestens auf einer Schlaglänge oder einem Mehrfachen der Schlaglänge fotografiert wird und dadurch erzeugte Aufnahmen aufeinanderfolgend wiedergegeben werden.

Sie betrifft ferner eine Vorrichtung zum Durchführen des Verfahrens.

Außerdem betrifft die Erfindung ein Verfahren zum Inspizieren eines laufenden Drahtseils, bei dem das laufende Drahtseil an einer ortsfesten Stelle in zeitlichen Abständen mindestens auf einer Schlaglänge oder einem Mehrfachen der Schlaglänge mit Lichtblitzen beleuchtet wird.

Aus der Veröffentlichung WEHKING K-H ET AL: "INNOVATIVE PROCEDURE FOR VISUAL ROPE INSPECTION", LIFT REPORT, VFZ VERLAG, DORTMUND, DE, Bd. 29, Nr. 3, 1. Januar 2003 (2003-01-01), Seiten 10,12,14, XP001162513, ISSN: 0341-3721 gehen eine Vorrichtung und ein Verfahren der eingangs genannten Art hervor. Mittels vier Kameras werden von einem laufenden Drahtseil Bildsequenzen erstellt und gespeichert. Die so aufgenommenen Bilder werden zur visuellen Inspektion auf einem Bildschirm wiedergegeben und lassen sich in verschiedenen Funktionen, z.B. Standbild, Vorlauf und Rücklauf, aufeinanderfolgend wiedergeben.

Ein weiteres Verfahren zur Seilinspektion ist aus der EP 0 271 728 A1 bekannt. Zur Messung und Überwachung von Eigenschaften eines Garns oder Seils werden mit Hilfe eines Bildsensors aufeinanderfolgende Abschnitte des Garns oder des Seils fotografiert und in elektrische Bildsignale umgesetzt. Die Bildsignale werden punktweise digitalisiert und in einem Bildsignalspeicher an den Bildpunkten zugeordneten Speicherplätzen gespeichert. Durch eine Recheneinheit werden die Werte der erfassten Eigenschaften aus den gespeicherten digitalen Bildsignalen ermittelt. Bei der Überwachung werden die aus mehrere nacheinander aufgenommenen Bilder eines Garns oder Seils erhaltenen Messwerte miteinander verglichen.

Aus der US 3,761,177 A ist eine Stroboskopvorrichtung zur Überwachung von auf Gewebebahnen aufgebrachten und sich regelmäßig wiederholenden Strukturen wie z.B. Drucken bekannt. Die fortlaufende Gewebebahn wird derart mit der Stroboskopvorrichtung beleuchtet, dass die Strukturen aufgrund des Stroboskopeffekts für einen Beobachter stationär erscheinen.

Aus der US 3,599,223 A ist eine Vorrichtung zur Überwachung von Fäden und Garnen auf Defekte bekannt. Die Vorrichtung zählt automatisch die Anzahl von Fehlern in den Fäden oder Garnen und fotografiert die Fehler.

Drahtseile sollen an sich täglich visuell auf Drahtbrüche und das Entstehen anderer Fehler untersucht werden. Praktisch ist das nicht durchführbar. Das Drahtseil muss für die Untersuchung stehen oder sehr langsam laufen, die Arbeit muss unterbrochen werden.

Magnetinduktive Drahtseilprüfung ist an laufenden Seilen möglich. Sie ist jedoch aufwendig.

Der Erfindung liegt die Aufgabe zugrunde, ein Verfahren bzw. eine Vorrichtung zu schaffen, mit denen sich ein laufendes Drahtseil einfacher als mit dem bekannten Stand der Technik inspizieren lässt.

Diese Aufgabe wird mit den alternativen Verfahren nach Anspruch 1 oder nach Anspruch 2 bzw. mit der Vorrichtung nach Anspruch 10 gelöst. Gemäß der Erfindung ist hinsichtlich des oben zuerst genannten Verfahrens vorgesehen, das Drahtseil in den zeitlichen Abständen, die gleich dem Quotienten aus der Schlaglänge bzw. dem Mehrfachen der Schlaglänge und der Laufgeschwindigkeit des Drahtseils sind, derart fotografiert wird, dass gewundene Litzen des Drahtseils in den Aufnahmen an der gleichen Stelle des Seilumfangs erscheinen, die so erzeugten Aufnahmen bei der Wiedergabe miteinander verglichen werden und ein durch die Wiedergabe der Aufnahmen entstehendes Bild auf Änderungen überwacht wird, die Schäden anzeigen.

Hinsichtlich des weiteren genannten Verfahrens ist vorgesehen, dass die zeitlichen Abstände gleich dem Quotienten aus der Schlaglänge oder einem Mehrfachen der Schlaglänge und der Laufgeschwindigkeit des Drahtseils sind und das Seil in einer derartigen Frequenz beleuchtet wird, dass die nacheinander beleuchteten Abschnitte des Drahtseils mit dem Auge als ununterbrochenes Bild wahrgenommen werden und das Bild auf Änderungen überwacht wird, die Schäden anzeigen.

Die gewundenen Litzen eines Drahtseils erscheinen nach einer Schlaglänge wieder an der gleichen Stelle des Seilumfangs. Aufeinanderfolgende Ausschnitte des Drahtseils von der Größe der Schlaglänge zeigen daher das gleiche Litzenbild, jede Litze liegt wieder an derselben Stelle. Das gilt dann ebenso für Ausschnitte, deren Größe ein Mehrfaches der Schlaglänge beträgt.
Das Drahtbild innerhalb der Litzen verschiebt sich dabei in der Regel. Es bleibt nur dann genau gleich, d.h. jeder Draht liegt wieder an derselben Stelle, wenn die Schlaglänge der Drähte in der Litze in einem bestimmten Verhältnis zur Schlaglänge der Litzen im Seil stehen. Es bleibt scheinbar gleich, wenn sich die Drähte um genau eine Drahtdicke oder ein ganzzahliges Vielfaches der Drahtdicke verschieben.

Wird ein laufendes Drahtseil von einer ortsfest angeordneten Kamera oder einem ortsfest angeordneten Stroboskop immer dann fotografiert bzw. angeblitzt, wenn genau eine Schlaglänge oder ein Mehrfaches der Schlaglänge vorbeigelaufen ist, so ist das fotografierte bzw. das durch die Lichtblitze vor ausreichend dunklerem Hintergrund sichtbar gemachte Bild immer das gleiche. Von einer bestimmten Frequenz ab (Bildfrequenz etwa 24 pro Sekunde) verschwimmen die Bilder vor dem Auge zu einem ununterbrochenen Bild.
Es entsteht ein "stehendes Bild" der Litzen. Die Drähte innerhalb der Litzen "wandern" in der Regel langsam, die Litzen scheinen sich um ihre eigene Achse zu drehen.
Schäden in dem Drahtseil verursachen eine blitzschnell vorübergehende, meist nicht bildlich erfassbare, aber wahrnehmbare Veränderung oder eine sichtbare Änderung der Wanderungsbewegung der Drähte.

Beim Betrachten des gleichbleibenden bzw. langsam wandernden Bildes ermüdet das Auge nicht. Wird eine Veränderung wahrgenommen, so wird der damit entdeckte Fehler später näher untersucht.

Die Variante der Blitzbeleuchtungen ist vor allem für sofortige, unmittelbare Betrachtung mit dem Auge vorgesehen.
Die Variante des Fotografierens erlaubt später gleichfalls die Betrachtung mit dem Auge.
Sie lässt sich aber auch, von teilweise bis hin zu vollständig, automatisieren.

Nach einer vorteilhaften Ausgestaltung der Erfindung wird die jeweilige Wiederkehr derselben äußeren Litze des laufenden Drahtseils an derselben Stelle erfasst und jede oder jede zweite oder dritte Wiederkehr zum Auslösen der Aufnahme bzw. des Lichtblitzes benutzt.
Damit wird auf einfache Weise jeweils der richtige Zeitpunkt für die nächste Aufnahme bzw. Blitzbeleuchtung sichergestellt, auch wenn sich die Zeitabstände beim Anfahren und beim Abbremsen des Drahtseils oder aus anderen Gründen, wie gewissen Schlaglängenänderungen über die Länge eines hängenden Seiles hinweg, ändern.
Grundsätzlich bestehen jedoch auch andere Möglichkeiten. Beispielsweise kann die Laufgeschwindigkeit des Drahtseils an dem Antrieb der Treibscheibe des Seiles abgenommen werden und der genannte Quotient von einem Computer ständig neu berechnet werden und die Zeitfolge der Aufnahmen bzw. Lichtblitze entsprechend gesteuert werden. Ändert sich auch die Schlaglänge, so kann dies mit einem gesonderten Schlaglängengeber erfasst und zur Berechnung des Quotienten mit eingegeben werden.

Zweckmäßigerweise wird die Wiederkehr der Litze durch Erfassen aller Litzen, vorzugsweise mittels eines auf die Litzenbuckel ansprechenden Näherungssensors, und Zählen der Litzen erfasst. D.h., dass beispielsweise bei sechs Litzen in der äußeren Lage jeder sechste Litzenbuckel zu derselben Litze gehört und die Aufnahme bzw. den Lichtblitz auslöst. Wenn gleichzeitig der Seilvorschub zwischen der Wiederkehr derselben Litze gemessen wird, kann diese Information zur Feststellung einer etwaigen Schlaglöngenverönderung in Abhängigkeit von der Seillänge genutzt werden. Hierbei kann es vorteilhaft sein, die Wiederkehr derselben Litze mit Hilfe mehrerer, versetzt angeordneter Sensoren zu erfassen. Auf diese Weise lässt sich feststellen, ob z.B. eine Verkürzung der Abstände zwischen zwei aufeinanderfolgenden Wahrnehmungen derselben Litze durch eine tatsächliche Verkürzung der Seilschlaglänge oder durch eine Verdrehung des Seiles zwischen den Sensorpositionen erfolgt ist.

Nach einer weiteren Ausgestaltung der Erfindung wird die Wiederkehr der Litze durch Erfassen einer Markierung der Litze erfasst.
Die Markierung kann beispielsweise optisch sein, z.B. aus einer Verkupferung bestehen, oder aus einer Magnetisierung bestehen oder eine radioaktive Markierung sein. Die Erfindung erstreckt sich insofern auch auf die Herstellung von Drahtseilen, die von vornherein für ihre späteren Inspektionen hergerichtet sind.

Das gilt auch für den weiteren Vorschlag, jeweils einen Draht der Außenlitzen sichtbar zu markieren, um das Wandern der Drähte in den Litzen besser sichtbar zu machen.

Nach einer weiteren Ausgestaltung der Erfindung wird die Erfassung der Wiederkehr derselben Litze des laufenden Drahtseils ferner dazu benutzt, durch Registrierung der Wiederkehr der Litze oder Registrierung der Buckel zu verfolgen, welche Stelle des Drahtseils sich jeweils an der genannten ortsfesten Stelle befindet, und davon ausgehend die Lage von Schadensstellen an dem Drahtseil zu registrieren.
Auch dies wäre jedoch mittels eines gesonderten Weggebers möglich.
Die Schadensstellen können dann später genauer untersucht werden.

Die in dem Bild erfasste Länge des Drahtseils braucht nicht auf eine Schlaglänge bzw. das genannte Mehrfache der Schlaglänge begrenzt zu werden. Ist sie größer, so kann ein Fehler zwar zwei mal in dem Bild erscheinen. Das muss aber zu keiner Irritierung, sondern kann sogar zu einer Verstärkung der Wahrnehmung führen.

Eine vorteilhafte Ausgestaltung der Verfahrensweise besteht darin, dass aufgrund einer in dem erfassten Bild wahrgenommenen Änderung die betreffende Schadensstelle an einer in Laufrichtung des Drahtseils hinter der genannten ortsfesten Stelle liegenden Stelle mit einer hochauflösenden Kamera fotografiert wird.
Bei unmittelbarer visueller Untersuchung kann man ggf. das Drahtseil auch sofort zurücklaufen lassen und die Schadensstelle suchen und untersuchen.

In der Regel wird man das Verfahren von verschiedenen Seiten her gleichzeitig durchführen, um den gesamten Seilumfang zu erfassen.

Eine Vorrichtung zum Durchführen des Verfahrens weist in einer von dem Drahtseil durchlaufenen Station eine Kamera auf, die auf das Drahtseil gerichtet ist und mit einer Steuerung verbunden ist; an die Kamera ist eine Auswertungs- und Registrierungseinrichtung angeschlossen.
In der Regel ist die Kamera oder Fotozelle mehrfach vorhanden und von verschiedenen Seiten auf das Drahtseil gerichtet, um den Umfang des Drahtseils vollständig zu erfassen.

Vorzugsweise sind die Steuerung und die Auswertungs- und Registriereinrichtung für die verschiedenen Kameras oder Fotozellen gemeinsam.

Schließlich ist in einer dritten Version der Erfindung vorgesehen, dass das laufende Drahtseil auf einer großen Länge fotografiert wird und die Aufnahme in wiederkehrende Längeneinheiten z.B. von der Größe einer Schlaglänge oder eines Mehrfachen der Schlaglänge zerlegt wird und die aufeinanderfolgenden Längeneinheiten verglichen werden und auf Änderungen in dem Bild untersucht werden, die Schäden anzeigen.
Die Zerlegung, nach Möglichkeit einer Aufnahme des gesamten Drahtseils, in die Längeneinheiten kann in einem manuellen Verfahren oder, bevorzugt, automatisch mit Hilfe eines Computers vorgenommen werden. Die Längeneinheiten können dann weiter in der gleichen Weise verarbeitet werden, als seien sie wie oben beschrieben durch wiederkehrendes Auslösen der Kamera direkt als Bildfolge entstanden.

Zur Herstellung eines, vorzugsweise digitalen, Bildes in der Breite des Seildurchmessers und in der Länge des gesamten Seiles wird z.B. die gleiche Technik der Belichtung durch eine schlitzförmige Blende angewandt, wie sie bei der Aufnahme von analogen oder digitalen Panoramafotos Verwendung findet. Es unterbleibt lediglich das Schwenken der Kamera oder der Linse; die Veränderung des durch den Schlitz abgebildeten Bildausschnitts erfolgt durch den Lauf des Drahtseiles selbst. Die Geschwindigkeit der Kamera muss nur mit der Seilgeschwindigkeit synchronisiert werden.

Im folgenden sei die Erfindung anhand von Zeichnungen weiter verdeutlicht.
- Fig. 1: zeigt einen Ausschnitt aus einem Drahtseil,
- Fig. 2: zeigt einen Ausschnitt aus Fig. 1,
- Fig. 3: zeigt einen Fig. 2 entsprechenden zweiten Ausschnitt,
- Fig. 4: zeigt einen Fig. 2 entsprechenden dritten Ausschnitt,
- Fig. 5: zeigt einen Fig. 2 entsprechenden vierten Ausschnitt und
- Fig. 6: zeigt als ein Ausführungsbeispiel schematisch eine Vorrichtung zum Inspizieren eines laufenden Drahtseils.

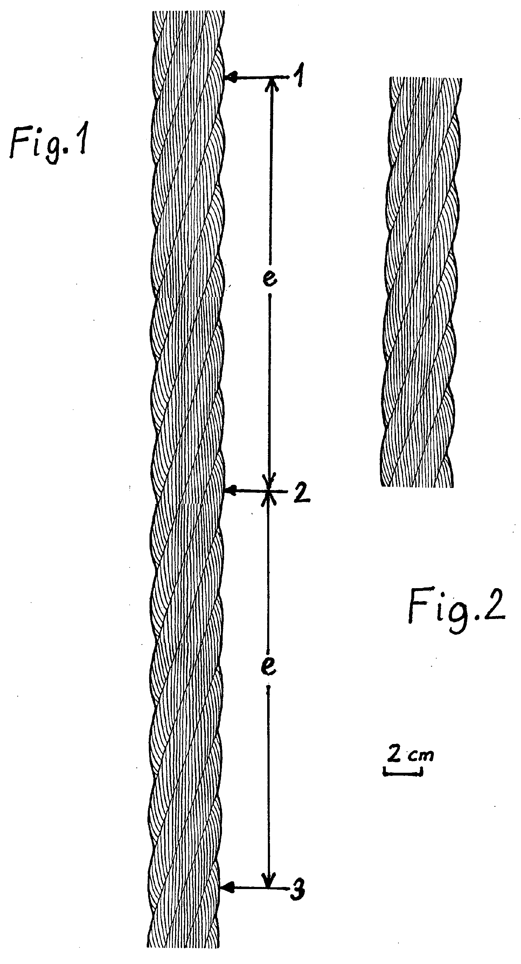

In Fig. 1 weisen die Pfeile 1, 2 und 3 auf das aufeinanderfolgende Auftreten derselben Litze an der jeweils um die Schlaglänge I versetzten gleichen Stelle des Seilumfangs eines Drahtseils 10 hin.
Zwischen den Pfeilen 1 und 2 und zwischen den Pfeilen 2 und 3 sind dieselben Litzen in derselben Konfiguration sichtbar.
Diese Konfiguration ist für sich dargestellt in Fig. 2.

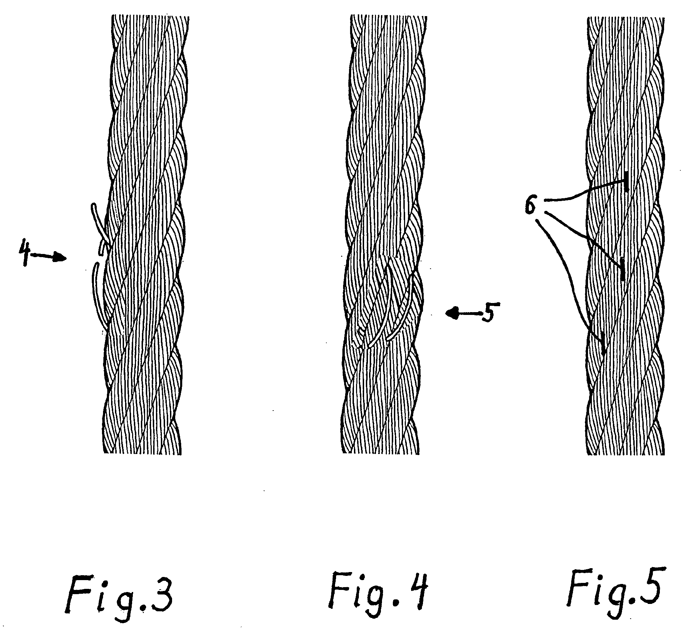

Sie ist jeweils noch einmal dargestellt in Fig. 3, 4 und 5, dort mit Drahtbrüchen 4 bzw. 5 bzw. 6. Die Drahtbrüche 4 und 5 sind übertrieben dargestellt. An den Drahtbrüchen 6 hat sich der Draht nur etwas zurückgezogen und eine Lücke gelassen, die sich mit Schmutz und Schmiermittel gefüllt hat und als kurzer dunkler Strich zu erkennen ist.

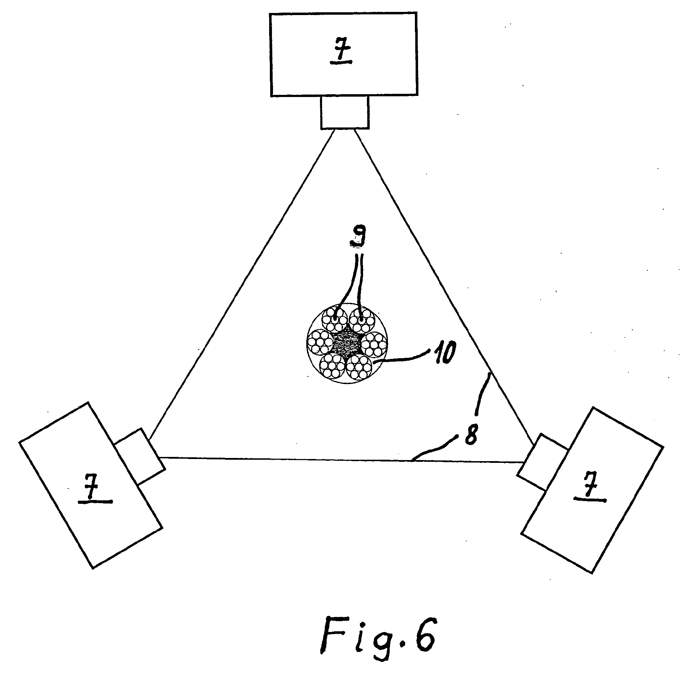

Fig. 6 zeigt eine Station mit drei von verschiedenen Seiten auf das Drahtseil 10 gerichtete Kamera 7. Linien 8 markieren die von den Kameras 7 erfassten Bereiche.

Die Schlaglänge der Litzen 9 des Drahtseils 10 beträgt im vorliegenden Beispiel 250 mm , die Laufgeschwindigkeit des Drahtseils 10 senkrecht zur Zeichenebene 5 m/sek und die Blitzfrequenz der Kameras 20 Hertz. Das heißt, das Drahtseil wird in Abständen von 0,05 sek. fotografiert, immer dann, wenn sich der nächste Abschnitt des Drahtseils von der Größe der Schlaglänge vor den Kameras 7 befindet.

Im übrigen kann auf die weiter oben gegebenen Erläuterungen verwiesen werden.

## Patentansprüche

1. Verfahren zum Inspizieren eines laufenden Drahtseils, bei dem das laufende Drahtseil an einer ortsfesten Stelle in zeitlichen Abständen mindestens auf einer Schlaglänge oder einem Mehrfachen der Schlaglänge fotografiert wird und dadurch erzeugte Aufnahmen aufeinanderfolgend wiedergegeben werden,
**dadurch gekennzeichnet,**
**dass** das Drahtseil in den zeitlichen Abständen, die gleich dem Quotienten aus der Schlaglänge bzw. dem Mehrfachen der Schlaglänge und der Laufgeschwindigkeit des Drahtseils sind, derart fotografiert wird, dass gewundene Litzen des Drahtseils in den Aufnahmen an der gleichen Stelle des Seilumfangs erscheinen, die so erzeugten Aufnahmen bei der Wiedergabe miteinander verglichen werden und ein durch die Wiedergabe der Aufnahmen entstehendes Bild auf Änderungen überwacht wird, die Schäden anzeigen.

2. Verfahren zum Inspizieren eines laufenden Drahtseils, bei dem das laufende Drahtseil an einer ortsfesten Stelle in zeitlichen Abständen mindestens auf einer Schlaglänge oder einem Mehrfachen der Schlaglänge mit Lichtblitzen beleuchtet wird,
**dadurch gekennzeichnet,**
**dass** die zeitlichen Abstände gleich dem Quotienten aus der Schlaglänge oder einem Mehrfachen der Schlaglänge und der Laufgeschwindigkeit des Drahtseils sind und das Seil in einer derartigen Frequenz beleuchtet wird, dass die nacheinander beleuchteten Abschnitte des Drahtseils mit dem Auge als ununterbrochenes Bild wahrgenommen werden und das Bild auf Änderungen überwacht wird, die Schäden anzeigen.

3. Verfahren nach Anspruch 1 oder 2,
**dadurch gekennzeichnet,**
**dass** die jeweilige Wiederkehr derselben gewundenen Litze des laufenden Drahtseils an demselben Ort erfasst und jede oder jede zweite oder dritte erfasste Wiederkehr zum Auslösen der Aufnahme bzw. des Lichtblitzes benutzt wird.

4. Verfahren nach Anspruch 3,
**dadurch gekennzeichnet,**
**dass** die Wiederkehr der Litze durch Erfassen aller Litzen, vorzugsweise mittels eines auf die Litzenbuckel ansprechenden Näherungssensors, und Zählen der Litzen erfasst wird.

5. Verfahren nach einem der Anspruch 3,
**dadurch gekennzeichnet,**
**dass** die Wiederkehr der Litze durch Erfassen einer Markierung der Litze erfasst wird.

6. Verfahren nach einem der Ansprüche 3 bis 5,
**dadurch gekennzeichnet,**
**dass** durch Registrierung der Wiederkehr der Litze oder Registrierung der Buckel verfolgt wird, welche Stelle des Drahtseils sich jeweils an der genannten ortsfesten Stelle befindet, und davon ausgehend die Lage von Schadensstellen an dem Drahtseil registriert wird.

7. Verfahren nach einem der Ansprüche 1 bis 5,
**dadurch gekennzeichnet,**
**dass** es zur Inspizierung von Drahtseilen vorgesehen ist, in denen jeweils ein Draht von Außenlitzen des Drahtseils sichtbar markiert ist.

8. Verfahren nach einem der Ansprüche 1 bis 7,
**dadurch gekennzeichnet,**
**dass** aufgrund einer in dem erfassten Bild wahrgenommenen Änderung die betreffende Schadensstelle an einer in Laufrichtung des Drahtseils hinter der genannten ortsfesten Stelle liegenden Stelle fotografiert wird.

9. Verfahren nach einem der Ansprüche 1 bis 8,
**dadurch gekennzeichnet,**
**dass** es von verschiedenen Seiten her gleichzeitig durchgeführt wird.

10. Vorrichtung zum Durchführen eines Verfahrens nach einem der Ansprüche 1 oder 3 bis 9 wenn abhängig vom Anspruch 1, die eine von dem Drahtseil (10) durchlaufene Station umfasst, in der eine Kamera (7) auf das Drahtseil (10) gerichtet ist, die mit einer Steuerung verbunden ist und an die eine Auswertungs- und Registriereinrichtung angeschlossen ist,
**dadurch gekennzeichnet,**
**dass** die Steuerung eingerichtet ist zur Steuerung der Kamera (7) derart, dass das laufende Drahtseil (10) auf einer Schlaglänge oder einem Mehrfachen der Schlaglänge in zeitlichen Abständen fotografiert wird, die gleich dem Quotienten aus der Schlaglänge bzw. dem Mehrfachen der Schlaglänge und der Laufgeschwindigkeit des Drahtseils (10) sind.

11. Vorrichtung nach Anspruch 10,
**dadurch gekennzeichnet,**
**dass** die Kamera (7) mehrfach vorhanden und von verschiedenen Seiten auf das Drahtseil (10) gerichtet ist.

12. Vorrichtung nach Anspruch 11,
**dadurch gekennzeichnet,**
**dass** die Steuerung und die Auswertungs- und Registriereinrichtung für die verschiedenen Kameras (7) gemeinsam sind.

## Claims

1. Method for inspecting a traveling wire cable, in which method the traveling wire cable is photographed at a stationary position at time intervals at least on one lay length or a multiple of the lay length, and pictures generated thereby are successively reproduced,
**characterized**
**in that** the wire cable is photographed at those time intervals which are equal to the quotient of the lay length, or a multiple of the lay length, and the travel speed of the wire cable in such a way that wound strands of the wire cable appear in the pictures at the same position of the cable circumference, the pictures produced thus are compared with one another when reproduced and an image arising by the reproduction of the pictures is monitored for changes that indicate damage.

2. Method for inspecting a traveling wire cable, in which method the traveling wire cable is illuminated with light flashes at a stationary position at time intervals at least on a lay length or a multiple of the lay length,
**characterized**
**in that** the time intervals are equal to the quotient of the lay length, or a multiple of the lay length, and the travel speed of the wire cable and the cable is illuminated with such a frequency that the successively illuminated portions of the wire cable are perceived by the eye as uninterrupted image and the image is monitored for changes that indicate damage.

3. Method according to Claim 1 or 2, **characterized in that** the respective return of the same wound strand of the traveling wire cable at the same place is detected, and each, or each second or third, detected return is used to trigger shooting or the light flash.

4. Method according to Claim 3, **characterized in that** the return of the strand is acquired by detecting all the strands, preferably by means of a proximity sensor responding to the strand bulge, and by counting the strands.

5. Method according to one of Claim 3, **characterized in that** the return of the strand is acquired by detecting a marking of the strand.

6. Method according to one of Claims 3 to 5, **characterized in that** recording the return of the strand, or recording the bulge, tracks the position of the wire cable that is respectively located at said stationary position, and the location of damaged positions on the wire cable is recorded starting therefrom.

7. Method according to one of Claims 1 to 5, **characterized in that** it is provided for inspecting wire cables in which respectively one wire of outer strands of the wire cable is visibly marked.

8. Method according to one of Claims 1 to 7, **characterized in that** on the basis of a change perceived in the acquired image the relevant damaged position is photographed at a position lying downstream of said stationary position in the travel direction of the wire cable.

9. Method according to one of Claims 1 to 8, **characterized in that** it is carried out simultaneously from various sides.

10. Device for carrying out a method according to one of Claims 1 or 3 to 9 when dependent on Claim 1, which device comprises a station traversed by the wire cable (10), in which station a camera (7) is directed onto the wire cable (10), which camera is connected to a controller, and to which camera an evaluation and recording device is connected,
**characterized**
**in that** the controller is configured to control the camera (7) in such a way that the traveling wire cable (10) is photographed at a lay length, or a multiple of the lay length, at time intervals which equal the quotient of the lay length, or a multiple of the lay length, and the travel speed of the wire cable (10).

11. Device according to Claim 10, **characterized in that** the camera (7) is multiply present and is directed onto the wire cable (10) from various sides.

12. Device according to Claim 11, **characterized in that** the controller and the evaluation and recording device are common to the various cameras (7).

## Revendications

1. Procédé pour inspecter un câble métallique en mouvement, dans lequel le câble métallique en mouvement est photographié à un emplacement stationnaire à des intervalles temporels au moins à raison d'un pas de câblage ou à raison d'un multiple du pas de câblage, et les prises de vue ainsi produites sont reproduites successivement,
**caractérisé en ce que**
le câble métallique est photographié à des intervalles temporels égaux au quotient du pas de câblage ou du multiple du pas de câblage et de la vitesse de mouvement du câble métallique, de telle sorte que des torons sinueux du câble métallique apparaissent au même endroit du pourtour de câble dans les prises de vue, les prises de vue ainsi produites sont comparées entre elles lors de la reproduction et une image qui se produit par la reproduction des prises de vue est surveillée quant à des modifications qui indiquent des dommages.

2. Procédé pour inspecter un câble métallique en mouvement, dans lequel le câble métallique est illuminé par des flashs lumineux à un emplacement stationnaire à des intervalles temporels au moins à raison d'un pas de câblage ou à raison d'un multiple du pas de câblage,
**caractérisé en ce que**
les intervalles temporels sont égaux au quotient du pas de câblage ou du multiple du pas de câblage et de la vitesse de mouvement du câble métallique et le câble est illuminé à une fréquence telle que les portions du câble métallique illuminées successivement sont perçues par l'oeil comme une image ininterrompue et que l'image est surveillée quant à des modifications qui indiquent des dommages.

3. Procédé selon la revendication 1 ou 2,
**caractérisé en ce que**
la récurrence respective du même toron sinueux du câble métallique en mouvement est détectée au même endroit, et chaque récurrence détectée ou une récurrence détectée sur deux ou sur trois est utilisée pour déclencher la prise de vue ou le flash lumineux.

4. Procédé selon la revendication 3,
**caractérisé en ce que**
la récurrence du toron est détectée en détectant tous les torons, de préférence au moyen d'un capteur de proximité qui réagit à des bossages du toron et en comptant les torons.

5. Procédé selon la revendication 3,
**caractérisé en ce que** la récurrence des torons est détectée en détectant un marquage.

6. Procédé selon l'une des revendications 3 à 5,
**caractérisé en ce que**
en enregistrant la récurrence du toron ou en enregistrant les bossages, on poursuit l'emplacement du câble métallique qui se trouve respectivement audit emplacement stationnaire, et à partir de ceci on enregistre la position d'emplacements endommagés sur le câble métallique.

7. Procédé selon l'une des revendications 1 à 5,
**caractérisé en ce que**
il est prévu pour inspecter des câbles métalliques dans lesquels un fil de torons extérieurs du câble métallique est marqué de façon visible.

8. Procédé selon l'une des revendications 1 à 7,
**caractérisé en ce que**
suite à une modification perçue dans l'image saisie, l'emplacement endommagé correspondant est photographié à un emplacement situé en arrière dudit emplacement stationnaire en direction de mouvement du câble métallique.

9. Procédé selon l'une des revendications 1 à 8,
**caractérisé en ce que**
il est mis en oeuvre simultanément depuis différents côtés.

10. Dispositif pour mettre en oeuvre un procédé selon l'une des revendications 1 ou 3 à 9 prise en dépendance de la revendication 1, qui comprend un poste traversé par le câble métallique (10), dans lequel une caméra (7) est dirigée sur le câble métallique (10), qui est connectée à une commande et à laquelle est branché un moyen d'évaluation et d'enregistrement,
**caractérisé en ce que**
la commande est conçue pour commander la caméra (7) de telle sorte que le câble métallique (10) en mouvement est photographié à raison d'un pas de câblage ou à raison d'un multiple du pas de câblage à des intervalles temporels qui sont égaux au quotient du pas de câblage ou du multiple du pas de câblage et de la vitesse de mouvement du câble métallique (10).

11. Dispositif selon la revendication 10,
**caractérisé en ce que**
la caméra (7) est présente plusieurs fois et est dirigée sur le câble métallique (10) depuis plusieurs côtés.

12. Dispositif selon la revendication 11,
**caractérisé en ce que**
la commande et le moyen d'évaluation et d'enregistrement sont en commun pour les différentes caméras (7).
